Europäisches Patentamt

European Patent Office

Office européen des brevets

(11) Veröffentlichungsnummer: **0 449 913 B1**

## (12) EUROPÄISCHE PATENTSCHRIFT

(45) Veröffentlichungstag der Patentschrift: **09.03.94**

(21) Anmeldenummer: **90900821.1**

(22) Anmeldetag: **15.12.89**

(86) Internationale Anmeldenummer:
**PCT/EP89/01548**

(87) Internationale Veröffentlichungsnummer:
**WO 90/07485 (12.07.90 90/16)**

(51) Int. Cl.⁵: **C07C 69/54**, C07C 67/08

---

(54) **VERFAHREN ZUR VERBESSERTEN HERSTELLUNG VON (METH)ACRYLSÄUREESTERN MEHRWERTIGER ALKOHOLE (III).**

---

(30) Priorität: **24.12.88 DE 3843930**

(43) Veröffentlichungstag der Anmeldung:
**09.10.91 Patentblatt 91/41**

(45) Bekanntmachung des Hinweises auf die
Patenterteilung:
**09.03.94 Patentblatt 94/10**

(84) Benannte Vertragsstaaten:
**BE CH DE ES FR GB IT LI NL**

(56) Entgegenhaltungen:
**FR-A- 2 316 253**

(73) Patentinhaber: **Henkel Kommanditgesellschaft auf Aktien**

**D-40191 Düsseldorf(DE)**

(72) Erfinder: **RITTER, Wolfgang**
**Am Bandenfeld 74**
**D-5657 Haan(DE)**
Erfinder: **SITZ, Hans-Dieter**
**Widdeshovener Strasse 48**
**D-4049 Rommerskirchen(DE)**
Erfinder: **SPEITKAMP, Ludwig**
**Tönisstrasse 13**
**D-4000 Düsseldorf 13(DE)**

(74) Vertreter: **von Kreisler, Alek, Dipl.-Chem. et al**
**Patentanwälte**
**von Kreisler-Selting-Werner**
**Postfach 10 22 41**
**D-50462 Köln (DE)**

---

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von polyfunktionellen Estern der Acrylsäure und/oder der Methacrylsäure mit mehrwertigen Alkoholen - im folgenden auch als (Meth)acrylsäureester bzw. Poly(Meth)acrylsäureester bezeichnet - durch Umsetzung der Reaktanten in Gegenwart saurer Veresterungskatalysatoren unter Zusatz von Polymerisationsinhibitoren zum Reaktionsgemisch.

(Meth)acrylsäureester mehrwertiger Alkohole insbesondere aus der Gruppe der 2- bis 4wertigen aliphatischen gesättigten Alkohole und deren Oxalkylierungsprodukte finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-Licht-härtenden Druckfarben oder entsprechenden Überzugslacken, Spachtel, Form- oder Vergußmassen sowie in Klebstoffen, insbesondere anaerob härtenden Klebstoffen Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)-acrylsäureester der hier betroffenen Art scheidet in aller Regel aufgrund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwiksamer Inhibitoren, die ihrerseits keine unerwünschten Nebenreaktionen, beispielsweise Verfärbungen, auslösen.

Zur Herstellung solcher polyfunktioneller (Meth)acrylsäureester mehrwertiger Alkohole besteht umfangreiche Vorliteratur. Verwiesen sei insbesondere auf die deutsche Offenlegungsschrift 29 13 218 und die darin zitierte einschlägige Literatur. So ist es aus der DE-AS 12 67 547 und aus der Zeitschrift "Chem. and Ind." 18 (1970), 597, bekannt, polyfunktionelle (Meth)acrylsäureester durch azeotrope Veresterung der (Meth)acrylsäure mit mehrwertigen Alkoholen in Gegenwart von azeotropen Schleppmitteln sowie von sauren Katalysatoren und Polymerisationsinhibitoren herzustellen. Als Polymerisationsinhibitoren wurden vorgeschlagen Phenole, Phenolderivate, Kupfer, Kupferverbindungen oder Phenothiazin. Als saure Katalysatoren werden organische oder anorganische Säuren oder saure Ionenaustauscher eingesetzt, wobei p-Toluolsulfonsäure und Schwefelsäure bevorzugt sein können. Die Veresterung erfolgt insbesondere bei Temperaturen von 40 bis 120 °C. Geeignete azeotrope Schleppmittel für die Entfernung des Reaktionswassers sind aliphatische oder cycloaliphatische oder aromatische Kohlenwssserstoffe bzw. deren Gemische mit Siedebereichen innerhalb der angegebenen Temperaturgrenzen.

In der genannten DE-OS 29 13 218 wird vorgeschlagen, die azeotrope Veresterung in Gegenwart mindestens eines organischen Esters der phosphorigen Säure zusätzlich zu einem mitverwendeten Inhibitor auf Phenolbasis durchzuführen. Zwingend wird allerdings auch hier die Mitverwendung mindestens eines aliphatischen, cycloaliphatischen und/oder aromatischen Kohlenwasserstoffs mit einem Siedepunkt im Bereich von 40 bis 120 °C gefordert. Das entstehende Reaktionswasser soll mittels dieser Schleppmittel azeotrop ausgekreist werden. Die Reaktionsdauer wird nach den Beispielen dieser Druckschrift mit 10 bis 18 Stunden angesetzt.

Die Erfindung geht von der Aufgabe aus, Reaktionsbedingungen für die hier betroffene Veresterungsreaktion zu ermitteln, die einerseits zu einer substantiellen Abkürzung der Reaktionsdauer führen können, andererseits aber die Qualität der entstehenden Veresterungsprodukte und insbesondere die hohe Farbqualität nicht negativ beeinflussen. Die Erfindung will weiterhin darauf verzichten können, vergleichsweise komplexe Inhibitorsysteme derart einsetzen zu müssen, wie sie in der genannten DE-OS 29 13 218 beschrieben sind. Es soll dabei erfindungsgemäß auch möglich sein, den im praktischen Einsatz gewünschten Applikationsinhibitor der hier betroffenen hochreaktiven Systeme gleichzeitig schon als Reaktionsinhibitor bei der Synthese der polyfunktionellen (Meth)acrylsäureester einzusehen.

Die Erfindung geht dabei unter anderem von der Erkenntnis aus, daß vergleichsweise hochreine Veresterungsprodukte als unmittelbare Verfahrensprodukte selbst dann erhalten werden können, wenn auf die Mitverwendung von Verdünnungsmitteln bzw. azeotropen Schleppmitteln verzichtet wird und daß es unter den Bedingungen des lösungsmittelfreien Arbeitens sogar möglich ist, vergleichsweise schärfere Veresterungsbedingungen einzusetzen, die zu einer beträchtlichen Abkürzung der Reaktionszeit führen. Voraussetzung hierfür ist insbesondere, daß der richtige Polymerisationsinhibitor gewählt und unter den nachfolgenden geschilderten Verfahrensbedingungen gearbeitet wird.

Gegenstand der Erfindung ist dementsprechend einVerfahren zur Herstellung von (Meth)-acrylsäureestern mehrwertiger Alkohole durch deren Umsetzung mit Acrylsäure und/oder Methacrylsäure in Gegenwart von sauren Veresterungskatalysatoren unter Zusatz von sterisch gehinderten phenolischen Verbindungen als Polymerisationsinhibitoren. Das neue Verfahren ist dadurch gekennzeichnet, daß man als sterisch gehinderte Phenolverbindungen Tocopherole und dabei bevorzugt wenigstens anteilsweise alpha-Tocopherol einsetzt.

Tocopherole als Polymerisationsinhibitoren sind in der Literatur gelegentlich schon vorgeschlagen worden. Ein praktischer Einsatz ist bisher jedoch nicht bekanntgeworden. Als Vitamin-E kommt dieser Verbindungsklasse vielmehr eine ganz andere praktische Anwendung zu. Die Erfindung geht von der überraschenden Erkenntnis aus, daß die mit der eingangs geschilderten Aufgabenstellung verbundenen besonderen Probleme durch die Verwendung gerade dieser Tocopherole und insbesondere des Alpha-Tocopherols wirkungsvoll gelöst werden können. Tatsächlich hat sich gezeigt, daß beim Einsatz dieser Inhibitoren verschärfte Verfahrensbedingungen eingesetzt werden können, wie sie bisher nicht als brauchbar angesehen wurden. So wird in der bevorzugten Ausführungsform des erfindungsgemäßen Verfahrens mit bei Reaktionstemperatur flüssigen Reaktionsgemischen gearbeitet, die wenigstens weitgehendfrei von Lösungs- und/oder azeotropen Schleppmitteln sind, wobei das Arbeiten in vollständiger Abwesenheit solcher flüssiger Hilfsmittel besonders bevorzugt ist. in einer weiterhin bevorzugten Ausführungsform des erfindungsgemäßen Verfahrens wird das bei der Veresterungsreaktion entstehende Kondensationswasser aus der Gasphase des Reaktionsraumes abgezogen.

Es ist erfindungsgemäß weiterhin bevorzugt, den Reaktionsraum mit einem Gasstrom zu durchspülen und diesen Gasstrom insbesondere dazu zu benutzen, das Kondensationswasser aus der Veresterungsreaktion aus dem Reaktor auszuschleusen. Bevorzugt wird dabei mit einem Gasstrom gearbeitet, der einen beschränkten Anteil an freiem Sauerstoff enthält. In Abhängigkeit von den jeweils gewählten Verfahrensbedingungen kann Luft oder ein an Sauerstoff verarmtes Gasgemisch eingesetzt werden. Ein Beispiel der zuletzt genannten Art sind Stickstoff/Luft-Gemische. In aller Regel wird allerdings ein gewisser Gehalt an freiem Sauerstoff in dieser der Reaktionsmischung zugeführten Gasphase gewünscht. Diese beschränkten Sauerstoffmengen aktivieren in an sich bekannter Weise den Inhibitor während des Reaktionsgeschehens.

Der Sauerstoffgehalt des Gasgemisches liegt im allgemeinen bei wenigstens etwa 1 Volumen-% und bevorzugt im Bereich von etwa 2 bis 20 Volumen-%. Aus Gründen der Reaktionssicherheit kann es dabei bevorzugt sein, mit Gehalten an freiem Sauerstoff in der unteren Hälfte des genannten Bereiches, also bis etwa 10 Volumen-% und bevorzugt bis etwa 7 Volumen-% zu arbeiten. Der Gasstrom wird in einer bevorzugten Ausführungsform der Erfindung in das flüssige Reaktionsgemisch eingespeist und kann dieses beispielsweise feinverteilt durchperlen. Es wird dabei zweckmäßigerweise mit begrenzten Mengen dieses Gasstromes gearbeitet, so daß kein unerwünscht hoher Austrag an Reaktionskomponenten - insbesondere der vergleichsweise leichter flüchtigen Säuren - stattfindet.

Der Polymerisationsinhibitor bzw. gegebenenfalls das inhibitorgemisch wird dem Reaktionsgemisch üblicherweise in Mengen von 200 bis 10000 ppm und bevorzugt im Bereich von etwa 300 bis 2000 ppm zugesetzt. Die Zahlenangaben beziehen sich dabei jeweils auf das Gewicht des aus (Meth)acrylsäure und polyfunktionellen Alkoholen bestehenden Reaktionsgemisches.

Als zu veresternde Polyalkohole seien beispielsweise genannt: Ethylenglycol, Propylenglycol, Butandiol-1,4, Hexandiol-1,6, Neopentylglycol, Diethylenglycol, Triethylenglycol, Dimethylolpropan, Glycerin, Trimethylolpropan, Trimethylolhexan, Trimethylolethan, Hexantriol-1,3,5 und Pentraerythrit. Erfindungsgemäß kommen als polyfunktionelle Alkohole insbesondere aber auch die Oxalkylierungsprodukte dieser zuvor genannten polyfunktionellen Alkohole in Betracht, wobei hier den Oxethylierungsprodukten und/oder den Oxpropylierungsprodukten besondere Bedeutung zukommt. Kettenverlängerte polyfunktionelle Alkohole dieser Art können beträchtliche Mengen an Polyalkoxidresten enthalten, beispielsweise 1 bis 50 Mol, vorzugsweise etwa 1 bis 20 Mol Ethylenoxid pro g-Äquivalent an Hydroxylgruppen.

Veresterungskatalysatoren für das erfindungsgemäße Herstellungsverfahren sind handelsübliche organische oder anorganische Säuren oder auch saure lonenaustauscher, wobei den in der Praxis häufig eingesetzten entsprechenden Verbindungen p-Toluolsulfonsäure und Schwefelsäure besondere Bedeutung zukommt. Die Mengen des Veresterungskatalysators liegen beispielsweise im Bereich von 0,1 bis 5 Gew.-% bezogen auf das Veresterungsgemisch.

Die Umsetzung der Reaktanten wird vorzugsweise bei Sumpftemperaturen von wenigstens etwa 90 °C und insbesondere von wenigstens etwa 100 °C durchgeführt. Besonders geeignet ist der Temperaturbereich bis etwa 150 °C. Dabei kann unter Normaldruck, zweckmäßigerweise aber auch unter abgesenktem Druck gearbeitet werden. Beim Arbeiten mit vermindertem Druck kann in einer besonderen Ausführungsform der Druck in Richtung auf niedrigere Drucke stufenweise oder auch kontinuierlich verringert werden.

Durch die Möglichkeit unter vergleichsweise scharfen Veresterungsbedingungen und gleichzeitig vermindertem Druck zu arbeiten, wird die Reaktionsdauer gegenüber bisher beschriebenen Verfahren stark abgekürzt. So können im erfindungsgemäßen Verfahren Umsatzausbeuten von wenigstens 90 % der Theorie und vorzugsweise von wenigstens etwa 94 % der Theorie im Temperaturbereich von etwa 100 bis 140 °C bei einer Reaktionsdauer von nicht mehr als etwa 10 Stunden und vorzugsweise von nicht mehr als etwa 8 Stunden erzielt werden. Gleichwohl fallen die Reaktionsprodukte als hellfarbige oder durch eine einfache Nachbehandlung wirkungsvoll zu reinigende stabilisierte Masse an. Das den sauren Veresterungskatalysator enthaltende Reaktionsrohprodukt wird einer nachfolgenden Neutralisation unterworfen. Diese Neutralisation kann unter bekannten Naßbedingungen, beispielsweise durch Einsatz von wäßrigen Soda- und gegebenenfalls Natriumchlorid enthaltenden Lösungen erfolgen. In einer bevorzugten Ausführungsform wird allerdings das den sauren Katalysator enthaltende Reaktionsrohprodukt einer trockenen Neutralisation unterworfen. Geeignete trockene Neutralisationsmittel sind die Oxide und/oder Hydroxide der Alkalimetalle, der Erdalkalimetalle und/oder des Aluminiums. Besonders geeignet sind zur Trockenneutralisation entsprechende Verbindungen des Magnesiums bzw. des Calciums.

(Meth)acrylsäure und die Alkohole können für die Veresterung in äquivalenten Menganverhältnissen eingesetzt werden. Bei den mehr als zweiwertigen Alkoholen ist es allerdings auch ohne weiteres möglich, nur einen Teil der Hydroxylgruppen zu verestern. Zur Vollveresterung kann es zweckmäßig sein, die Säurekomponente in leichtem Überschuß über die zur Veresterung der Hydroxylgruppen erforderliche stöchiometrische Menge einzusetzen. Ein solcher Überschuß kann wenigstens etwa 10 Mol-% ausmachen. Nach Abschluß der Reaktion kann gewünschtenfalls zusätzlich ein Inhibitor dem Reaktionsprodukt beigemischt werden.

Treten bei der Herstellung der Reaktionsprodukte unter den erfindungsgemäßen drastischen Veresterungsbedingungen dann doch einmal leichte Farbverschlechterungen im Reaktionsprodukt auf, so lassen sich diese problemlos durch eine Nachbehandlung mit Entfärbungsmitteln beseitigen. Ein geeignetes Entfärbungsmittel ist beispielsweise Aluminiumoxid.

## Beispiele

### Beispiel 1

324,0 g Acrylsäure, 368,2 g eines ethoxylierten Trimethylolpropans (OH-Zahl 680 mg KOH/g Substanz), 24,2 g p-Toluolsulfonsäure und 1,38 g D,L-alpha-Tocopherol (Fa. Merck, 2260 ppm bezogen auf die Produktmenge) wurden in einen 1-Liter-Dreihalskolben eingewogen.

Unter Durch leiten eines Luft/Stickstoff-Gemisches (5 Vol-% $O_2$; 40 l/h) wurde die Veresterung unter Wasserabtrennung durchgeführt. Bei einer konstanten Badtemperatur von 145 °C und einer maximalen Sumpftemperatur von 140 °C betrug die Veresterungszeit 4 Stunden.

Rohproduktdaten:

| | |
|---|---|
| Säurezahl: | 20,3 mg KOH/g |
| OH-Zahl: | 19,7 mg KOH/g |
| Umsatz: | 95,0 % |
| Gardner Farbzahl: | 7 - 8 |
| $H_2$O-Gehalt: | 0,12 % |

Das Rohprodukt wurde mit 700 ml 10 Gew.-%iger wäßriger Natriumcarbonat-Lösung gewaschen, im Vakuum bei 40 mbar und 80 °C 3 Stunden getrocknet und anschließend filtriert.

Produktdaten:

| | |
|---|---|
| Säurezahl: | < 1 mg KOH/g |
| OH-Zahl: | 43 mg KOH/g |
| Gardner Farbzahl: | 3 - 4 |
| $H_2$O-Gehalt: | 0,31 % |

### Beispiel 2

324,0 g Acrylsäure, 368,2 g eines ethoxylierten Trimethylolpropans (OH-Zahl 680 mg KOH/g Substanz), 24,2 g p-Toluolsulfonsäure wurden in einen 1-Liter-Kolben eingewogen und mit 1,22 g Alpha-Tocopherol (Fa. Henkel, 2000 ppm bezogen auf die Produktmenge) inhibiert. Unter Durchleiten eines Luft/StickstoffGemisches (5 Vol-% $O_2$; 40 l/h) wurde die Veresterung unter Wasserabtrennung durchgeführt. Bei einer konstanten Badtemperatur von 125 °C und einer maximalen Sumpftemperatur von 120 °C betrug bei einem Druck von 700 mbar die Veresterungszeit 4 Stunden.

Rohproduktdaten:

| | |
|---|---|
| Säurezahl: | 17,0 mg KOH/g |
| OH-Zahl: | 33,6 mg KOH/g |
| Umsatz: | 91,4 % |
| Gardner Farbzahl: | 7 - 8 |
| $H_2O$-Gehalt: | 0,16 % |

Das Rohprodukt wurde mit 1400 g wäßriger 16 Gew.-% NaCl/4 Gew.-% $NaHCO_3$-Lösung gewaschen, im Vakuum bei 40 mbar und 80 °C 2 Stunden getrocknet und anschließend filtriert.

Produktdaten:

| | |
|---|---|
| Säurezahl: | < 1 mg KOH/g |
| OH-Zahl: | 43 mg KOH/g |
| Gardner Farbzahl: | 5 - 6 |
| $H_2O$-Gehalt: | 0,42 % |

Beispiel 3

Beispiel 2 wurde wiederholt mit der Änderung, daß anstelle des 2000 ppm Alpha-Tocopherols 5000 ppm eines Misch-Tocopherols (Fa. Henkel) verwendet wurde.

Rohproduktdaten:

| | |
|---|---|
| Säurezahl: | 33,1 mg KOH/g |
| OH-Zahl: | 27,8 mg KOH/g |
| Umsatz: | 92,9 % |
| Gardner Farbzahl: | 12 |
| $H_2O$-Gehalt: | 0,18 % |

Das Rohprodukt wurde wie in Beispiel 1 aufgearbeitet.

Produktdaten:

| | |
|---|---|
| Säurezahl: | < 1 mg KOH/g |
| OH-Zahl: | 39 mg KOH/g |
| Gardner Farbzahl: | 5 - 6 |
| $H_2O$-Gehalt: | 0,25 % |

Beispiel 4

1559,5 g Acrylsäure, 1521,0 g eines ethoxylierten Trimethylolpropans (OH-Zahl 665 mg KOH/g Substanz) und 107,8 g p-Toluosulfonsäure würden in einen 3-Liter-Reaktor eingewogen und mit 4,12 g Alpha-Tocopherol (Fa. Henkel, 1650 ppm bezogen auf die Produktmenge) inhibiert. Unter Durchleiten von Luft (40 l/h) wurde die Veresterung unter Acrylsäureüberschuß-Abtrennung durchgeführt. Bei einer maximalen Sumpftemperatur von 105 °C betrug die Veresterungszeit 6 Stunden bei einem Druck von 400 mbar.

Rohproduktdaten

| | |
|---|---|
| Säurezahl: | 24,9 mg KOH/g |
| OH-Zahl: | 23,5 mg KOH/g |
| Umsatz: | 93,9 % |
| Gardner Farbzahl: | 11 |
| $H_2O$-Gehalt: | 0,25 % |

Das Rohprodukt wurde durch Zugabe von 102,1 g festen $Ca(OH)_2$ und 1,5-stündigem Rühren bei 80 °C und 50 mbar neutralisiert.

Produktdaten:

| | |
|---|---|
| Säurezahl: | < 1 mg KOH/g |
| OH-Zahl: | 26,5 mg KOH/g |
| Gardner Farbzahl: | 3 - 4 |
| $H_2O$-Gehalt: | 0,18 % |

Tabelle 1: Substanzveresterung mit Vitamin E Inhibierung

| Inhibitor/Inhibitormenge ppm | Reaktionsbedingungen | | Gardner Farbzahl des Produktes | | Umsatz *1 |
|---|---|---|---|---|---|
| | T °C | p mbar | | | % |
| DL-alpha-Tocopherol (Fa. Merck) | | | | | |
| 5000 | 140 | 1013 | 5-6 | *2 | 93,8 |
| 2260 | 140 | 1013 | 3-4 | *3 | 95,0 |
| Alpha-Tocopherol (Fa. Henkel) | | | | | |
| 10000 | 140 | 1013 | 7-8 | *3 | 93,1 |
| 5000 | 120 | 700 | 7-8 | *2 | 92,5 |
| 2000 | 120 | 700 | 5-6 | *2 | 91,4 |
| 1650 | 105 | 400 | 3-4 | *4 | 93,9 |
| 1000 | 105 | 400 | 4-5 | *4 | 92,5 |
| 500 | 105 | 400 | 4-5 | *4 | 93,3 |
| 300 | 105 | 400 | 3-4 | *4 | 93,1 |
| Misch-Tocopherol (Fa. Henkel) | | | | | |
| 10000 | 140 | 1013 | 9-10 | *2 | 91,3 |
| 5000 | 120 | 700 | 5-6 | *3 | 92,9 |
| 2000 | 120 | 700 | 5-6 | *3 | 93,8 |

*1   Umsatz bezogen auf die OH-Zahl

*2   Wäsche des Rohproduktes mit 4 Gew.-% Natriumhydrogencarbonat/16 Gew.-% Natriumchlorid wäßriger Lösung und anschließende Trocknung

*3    Wäsche    des    Rohproduktes    mit    10    Gew.-%
     Natriumcarbonat-Lösung und anschließende Trocknung

*4    Neutralisation des Rohproduktes mit festem Calciumhydroxid
     (2facher equimolarer Überschuß bezogen auf die Säurezahl)

Beispiel 5

1559,5 g Acrylsäure, 1521,0 g eines ethoxylierten Trimethylolpropans (OH-Zahl 665 mg KOH/g Substanz) und 107,8 g p-Toluolsulfonsäure wurden in einen 3-Liter-Reaktor eingewogen und mit 4,99 g Alpha-Tocopherol (Fa. Henkel, 2000 ppm bezogen auf die Produktmenge) inhibiert. Unter Durchleiten von Luft (40 l/h) wurde die Veresterung unter Acrylsäureüberschuß- und Wasser-Abtrennung durchgeführt. Bei einer Sumpftemperatur von 105 °C betrug die Veresterungszeit 6 Stunden bei einem Druck von 400 mbar.

Rohproduktdaten:

| Säurezahl: | 26,3 mg KOH/g |
| OH-Zahl: | 24,8 mg KOH/g |
| Umsatz: | 94,2 % |
| Gardner Farbzahl: | 10 |
| $H_2O$-Gehalt: | 0,18 % |

Das Rohprodukt wurde durch Zugabe von 107,8 g festem $Ca(OH)_2$ und 1,5-stündigem Rühren bei 80 °C und 50 mbar neutralisiert und anschließend mit einer Druckfilternutsche filtriert.

Produktdaten:

| Säurezahl: | < 1 mg KOH/g |
| OH-Zahl: | 27,0 mg KOH/g |
| Gardner Farbzahl: | 3 - 4 |
| $H_2O$-Gehalt: | 0,17 % |

Zur Verbesserung der Produktfarbe wurde das Produkt mit 240 g $Al_2O_3$ (basisch) 2 Stunden bei 80 °C gerührt und anschließend mit Hilfe einer Druckfilternutsche filtriert.

| Säurezahl: | < 1 mg KOH/g |
| OH-Zahl: | 28,0 mg KOH/g |
| Gardner Farbzahl: | < 1 |

Beispiel 6

1320,0 g Acrylsäure, 1861,7 g eines propoxylierten Neopentylglycols (OH-Zahl: 460 mg KOH/g Substanz) sowie 111,4 g p-Toluolsulfonsäure wurden in einen 3-Liter-Reaktor eingewogen und mit 5,37 g alpha-Tocopherol (Fa. Henkel, 2000 ppm bezogen auf die Produktmenge) inhibiert. Unter Durchleiten von Luft (40 l/h) wurde die Veresterung unter Wasser- und Acrylsäureüberschuß-Abtrennung durchgeführt. Bei einer Sumpftemperatur von 105 °C betrug die Veresterungszeit 6 Stunden bei einem Druck von 400 mbar.

Rohproduktdaten:

| | |
|---|---|
| Säurezahl: | 31,0 mg KOH/g |
| OH-Zahl: | 18,0 mg KOH/g |
| Umsatz: | 94,6 % |
| Gardner Farbzahl: | 8 |
| $H_2O$-Gehalt: | 0,21 % |

Das Rohprodukt wurde durch Zugabe von 114 g festem $Ca(OH)_2$ und 2-stündigem Rühren bei 80 °C und 50 mbar neutralisiert und anschließend mit Hilfe einer Druckfilternutsche filtriert.

Produktdaten:

| | |
|---|---|
| Säurezahl: | < 1 mg KOH/g |
| OH-Zahl: | 21,0 mg KOH/g |
| Gardner Farbzahl: | 3 |
| $H_2O$-Gehalt: | 0,17 % |

Zur Verbesserung der Produktfarbe wurde das Produkt mit 260 g $Al_2O_3$ (basisch) 2 Stunden bei 80 °C gerührt und anschließend mit Hilfe einer Druckfilternutsche filtriert.

| | |
|---|---|
| Säurezahl: | < 1 mg KOH/g |
| OH-Zahl: | 21,0 mg KOH/g |
| Gardner Farbzahl: | < 1 |

Tabelle 2

Entfärbungswirkung von $Al_2O_3$ (basisch) bei der Substanzveresterung mit Vitamin E-Inhibierung

Trimethylolpropan + 3 EO-triacrylat/Farbzahl vor der Reinigung Gardner 3 - 4

| $Al_2O_3$ Menge Gew.-% bez. auf Produktmenge | Gardner Farbzahl |
| --- | --- |
| 5 | 2 - 3 |
| 10 | 1 |
| 15 | < 1 |
| 20 | < 1 |

Neopentylglycol + 2 PO-diacrylat/Farbzahl vor der Reinigung Gardner 3

| 5 | 2 |
| --- | --- |
| 10 | 1 |
| 15 | < 1 |
| 20 | < 1 |

**Patentansprüche**

1. Verfahren zur Herstellung von (Meth)acrylsäureestern mehrwertiger Alkohole durch Umsetzung der Reaktanten in Gegenwart von sauren Veresterungskatalysatoren unter Zusatz von sterisch gehinderten Phenolverbindungen als Polymerisationsinhibitoren, dadurch gekennzeichnet, daß man als sterisch gehinderte Phenolverbindungen Tocopherole und dabei bevorzugt wenigstens anteilsweise alpha-Tocopherol einsetzt.

2. Verfahren nach Anspruch 1, dadurch gekennzeichnet, daß man mit bei Reaktionstemperatur flüssigen Reaktionsgemischen arbeitet, die wenigstens weitgehend frei sind von Lösungs- und/oder azeotropen Schleppmitteln und daß das entstehende Kondensationswasser aus der Gasphase des Reaktionsraumes abgezogen wird.

3. Verfahren nach Ansprüchen 1 und 2, dadurch gekennzeichne, daß man den Reaktionsraum mit einem freien Sauerstoff enthaltenden Gasstrom durchspült.

4. Verfahren nach Ansprüchen 1 bis 3, dadurch gekennzeichnet, daß man mit Luft oder einem an $O_2$-abgereicherten Gasgemisch, insbesondere mit Stickstoff/Luft-Gemischen arbeitet.

5. Verfahren nach Ansprüchen 1 bis 4, dadurch gekennzeichnet, daß die Veresterung bei Sumpftemperaturen von wenigstens etwa 90 °C, vorzugsweise von wenigstens etwa 100 °C und insbesondere im

EP 0 449 913 B1

Bereich bis etwa 150 °C durchgeführt wird, wobei bevorzugt wenigstens absatzweise bei vermindertem, gegebenenfalls stufenweise zunehmend vermindertem Druck, gearbeitet wird.

6. Verfahren nach Ansprüchen 1 bis 5, dadurch gekennzeichnet, daß die Inhibitoren in Mengen von 200 bis 10000 ppm, bevorzugt im Bereich von 300 bis 2000 ppm - jeweils bezogen auf das Gewicht des Reaktionsgemisches - eingesetzt werden.

7. Verfahren nach Ansprüchen 1 bis 6, dadurch gekennzeichnet, daß die Reaktion auf einen Umsatz von wenigstens 90 % der Theorie, vorzugsweise von wenigstens 94 % der Theorie geführt wird, wobei man bevorzugt im Temperaturbereich von etwa 100 bis 140 °C - gewünschtenfalls unter Vakuum - für eine Reaktionsdauer von nicht mehr als 10 Stunden, insbesondere von nicht mehr als 8 Stunden, arbeitet.

8. Verfahren nach Ansprüchen 1 bis 7, dadurch gekennzeichnet, daß das Reaktionsrohprodukt einer trockenen Neutralisation - bevorzugt mit Oxiden und/oder Hydroxiden der Alkalimetalle, der Erdalkalimetalle und/oder des Aluminiums - unterworfen wird.

9. Verfahren nach Ansprüchen 1 bis 8, dadurch gekennzeichnet, daß die primär anfallenden Reaktionsprodukte einer abschließenden Behandlung mit Entfärbungsmitteln unterworfen werden.

## Claims

1. A process for the production of (meth)acrylic acid esters of polyhydric alcohols by reaction of the reactants with acrylic acid and/or methacrylic acid in the presence of acidic esterification catalysts with addition of sterically hindered phenolic compounds as polymerization inhibitors, characterized in that tocopherols are used as the sterically hindered phenolic compounds and $\alpha$-tocopherol is preferably at least partly used.

2. A process as claimed in claim 1, characterized in that the reaction mixtures used are liquid at room temperature and are at least substantially free from solvents and/or azeotropic entraining agents and in that the water of condensation formed is removed from the gas phase of the reaction zone.

3. A process as claimed in claims 1 and 2, characterized in that the reaction zone is purged with a gas stream containing free oxygen.

4. A process as claimed in claims 1 to 3, characterized in that air or $O_2$-depleted gas mixtures, more particularly nitrogen/air mixtures, are used.

5. A process as claimed in claims 1 to 4, characterized in that the esterification reaction is carried out at sump temperatures of at least about 90°C, preferably of at least about 100°C and, more preferably, in the range up to about 150°C, the reaction preferably being carried out at least in batches under reduced pressure and optionally under a pressure increasingly reduced in steps.

6. A process as claimed in claims 1 to 5, characterized in that the inhibitors are used in quantities of from 200 to 10,000 ppm and preferably in quantities of from 300 to 2,000 ppm, based on the weight of the reaction mixture.

7. A process as claimed in claims 1 to 6, characterized in that the reaction is continued to a yield of at least 90% of the theoretical and preferably of at least 94% of the theoretical, preferably being carried out at a temperature in the range from about 100 to 140°C, optionally in vacuo, over a reaction time of no more than 10 hours and, in particular, no more than 8 hours.

8. A process as claimed in claims 1 to 7, characterized in that the crude reaction product is subjected to dry neutralization, preferably with oxides and/or hydroxides of the alkali metals, the alkaline earth metals and/or aluminium.

9. A process as claimed in claims 1 to 8, characterized in that the reaction products initially obtained are subjected to a final treatment with decolorizing agents.

11

**Revendications**

1. Procédé de préparation de (meth)acrylates de polyalcools par réaction des réactifs et en présence de catalyseurs acides d'estérification en présence de composés phénoliques stériquement gênés comme inhibiteurs de polymérisation, caractérisé en ce qu'on utilise comme composés phénoliques stériquement gênés des tocophérols et dans ce cas du moins en partie de l'alpha-tocophérol.

2. Procédé selon la revendication 1, caractérisé en ce qu'on travaille avec des mélanges réactionnels liquides à la température de réaction, qui sont au moins largement exempts de solvants et/ou d'agents d'entraînement azéotropiques et qu'on élimine de la phase gazeuse de la chambre réactionnelle l'eau de condensation fermée.

3. Procédé selon les revendications 1 et 2, caractérisé en ce qu'on balaie la chambre réactionnelle avec un courant gazeux contenant de l'oxygène libre.

4. Procédé selon les revendications 1 à 3, caractérisé en ce qu'on travaille avec de l'air ou un mélange gazeux appauvri en $O_2$, notamment des mélanges azote/air.

5. Procédé selon les revendications 1 à 4, caractérisé en ce qu'on réalise l'estérification à des températures de produit au moins d'environ 90°C, de préférence au moins d'environ 100°C et notamment dans l'intervalle jusqu'à environ 150°C, en travaillant de préférence au moins par intervalles sous pression réduite, le cas échéant par étapes et sous pression de plus en plus réduite.

6. Procédé selon les revendications 1 à 5, caractérisé en ce qu'on utilise des inhibiteurs à des concentrations de 200 à 10.000 ppm, de préférence dans l'intervalle de 300 à 2000 ppm à chaque fois rapporté au poids du mélange réactionnel.

7. Procédé selon les revendications 1 à 6, caractérisé en ce qu'on conduit la réaction jusqu'à un rendement d'au moins 90 % de la théorie, de préférence d'au moins 94 % de la théorie, en travaillant de préférence dans un intervalle de température d'environ 100 à 140°C, le cas échéant sous vide, pour une durée de réaction qui ne dépasse pas 10 heures, notamment qui ne dépasse pas 8 heures.

8. procédé selon les revendications 1 à 7, caractérisé en ce qu'on soumet le produit brut de réaction à une neutralisation à sec, de préférence avec des oxydes et/ou hydroxydes de métaux alcalins, de métaux alcalino-terreux et/ou d'aluminium.

9. Procédé selon les revendications 1 à 8, caractérisé en ce qu'on soumet les produits de reaction primaires à un traitement consécutif avec des agents décolorants.